# EUROPEAN PATENT APPLICATION

(11) **EP 4 491 604 A1**
(43) Date of publication of application: **15.01.2025**
(21) Application number: 24744718.8
(22) Date of filing: 18.01.2024
(51) Int. Cl.: C07C 21/18, C07C 21/22, C07C 23/04, C07C 53/02, C09K 5/04

(54) **COMPOSITION CONTAINING 1,2-DIFLUOROETHYLENE**

(30) Priority: 18.01.2023 JP 2023006036
(71) Applicant: DAIKIN INDUSTRIES, LTD., Osaka-shi, Osaka 530-0001 (JP)
(72) Inventor: TAKAHASHI, Kazuhiro, Osaka-Shi, Osaka 530-0001 (JP); GOTOU, Tomoyuki, Osaka-Shi, Osaka 530-0001 (JP); KARUBE, Daisuke, Osaka-Shi, Osaka 530-0001 (JP)
(74) Representative: Hoffmann Eitle
(86) International application number: PCT/JP2024/001331
(87) International publication number: WO 2024/154798

(57) **Abstract**

To provide a new composition containing 1,2-difluoroethylene (HFO-1132).

A composition containing: HFO-1132; and at least one compound selected from the group consisting of: a fluoroacetylene represented by CX*CR¹, wherein * is a triple bond, R¹ is a monovalent group represented by CₙX²ₘ, where n is 0, 1, or 2, m = 2n + 1, and X and X¹ are the same or different and are H or F, and at least one of X and X¹ is F; a carboxylic acid fluoride represented by R²COF, wherein R² is a monovalent group represented by CₒX²ₚ, where o is 0 or 1, p = 2o + 1, and X² is H or F; a cyclic alkane represented by C_{q}HᵣFₛ, wherein q is 3, 4, or 5, r and s are integers of 0 or more and 2q or less that are the same as or different from each other, and r + s < 2q; and formic acid.

## Description

### Technical Field

The present disclosure relates to a composition containing 1,2-difluoroethylene.

### Background Art

A composition containing 1,2-difluoroethylene and for use in cooling, air conditioning, and heat pump systems is known (PTL 1). Also known is a working medium for heat cycles characterized by containing 1,2-difluoroethylene (PTL 2).

### Citation List

### Patent Literature

PTL 1: U.S. Patent Application Publication No. 2011/0253927
PTL 2: WO2012/157765

### Summary of Invention

### Technical Problem

An object of the present disclosure is to provide a new composition containing 1,2-difluoroethylene.

### Solution to Problem

### Item 1.

A composition comprising:
1,2-difluoroethylene (HFO-1132); and
at least one compound selected from the group consisting of: a fluoroacetylene represented by CX*CR¹, wherein * is a triple bond, R¹ is a monovalent group represented by CₙX¹ₘ, where n is 0, 1, or 2, m = 2n + 1, and X and X¹ are the same or different and are H or F, and at least one of X and X¹ is F; a carboxylic acid fluoride represented by R²COF, wherein R² is a monovalent group represented by CₒX²ₚ, where o is 0 or 1, p = 2o + 1, and X² is H or F; a cyclic alkane represented by C_{q}HᵣFₛ, wherein q is 3, 4, or 5, r and s are integers of 0 or more and 2q or less that are the same as or different from each other, and r + s < 2q; and formic acid.

### Item 2.

The composition according to Item 1, comprising the compounds in a total amount of 0.1 mass% or less based on the composition.

### Item 3.

The composition according to Item 1 or 2, which is a heat transfer medium, a foaming agent, or a propellant.

### Advantageous Effects of Invention

The present disclosure provides a new composition containing 1,2-difluoroethylene.

### Description of Embodiments

### 1. Composition of Present Disclosure

The present disclosure discloses a composition containing:
1,2-difluoroethylene (HFO-1132); and
at least one compound (hereinafter, sometimes referred to as additional compound) selected from the group consisting of: a fluoroacetylene represented by CX*CR¹, wherein * is a triple bond, R¹ is a monovalent group represented by CₙX²ₘ, where n is 0, 1, or 2, m = 2n + 1, and X and X¹ are the same or different and are H or F, and at least one of X and X¹ is F; a carboxylic acid fluoride represented by R²COF, wherein R² is a monovalent group represented by CₒX²ₚ, where o is 0 or 1, p = 2o + 1, and X² is H or F; a cyclic alkane represented by C_{q}HᵣFₛ, wherein q is 3, 4, or 5, r and s are integers of 0 or more and 2q or less that are the same as or different from each other, and r + s < 2q; and formic acid.

As 1,2-difluoroethylene, there are trans-1,2-difluoroethylene (HFO-1132(E)) and cis-1,2-difluoroethylene (HFO-1132(Z)) isomers. Either one or a mixture of them is suitable for the composition of the present invention, but a composition containing trans-1,2-difluoroethylene (HFO-1132(E)) is preferable.

The composition according to the present disclosure at least contains, as an additional compound, at least one compound selected from the group consisting of:
an additional compound (1): a fluoroacetylene represented by CX*CR¹, wherein * is a triple bond, R¹ is a monovalent group represented by CₙX²ₘ, where n is 0, 1, or 2, m = 2n + 1, and X and X¹ are the same or different and are H or F, and at least one of X and X¹ is F;
an additional compound (2): a carboxylic acid fluoride represented by R²COF, wherein R² is a monovalent group represented by CₒX²ₚ, where o is 0 or 1, p = 2o + 1, and X² is H or F;
an additional compound (3): a cyclic alkane represented by C_{q}HᵣFₛ, wherein q is 3, 4, or 5, r and s are integers of 0 or more and 2q or less that are the same as or different from each other, and r + s < 2q; and
an additional compound (4): formic acid.

As the additional compound (1), one in which n is 0 or 1 in the above is preferable.

As the additional compound (1), one in which m is 1 or 3 in the above is preferable.

Specific examples of preferable additional compounds (1) include monofluoroacetylene, difluoroacetylene, and 3,3,3-trifluoroacetylene.

As the additional compound (2), one in which p is 0 to 3 in the above is preferable.

Specific examples of preferable additional compounds (2) include formyl fluoride, monofluoroacetic acid fluoride, difluoroacetic acid fluoride, and trifluoroacetic acid fluoride.

As the additional compound (3), one in which q is 3, 4, or 5, and is 3 or 4 in the above is preferable.

As the additional compound (3), one in which r is 0 to 10 in the above is preferable, and one in which r is 3 to 8 is more preferable.

As the additional compound (3), one in which s is 0 to 10 in the above is preferable, and one in which s is 3 to 8 is more preferable.

Specific examples of preferable additional compounds (3) include 1,2,3-trifluorocyclopropane, 1,2,3,4-tetrafluorocyclobutane, and perfluorocyclobutane.

In the present disclosure, a composition containing a specific amount of the above additional compounds is a stable composition of HFO-1132. Specifically, the amount of acid production over time in the case where moisture coexists is more suppressed, and/or the polymerization reaction under pressurization is more suppressed.

From the standpoint of the above stability improving effect, the composition according to the present disclosure contains the additional compounds in a total amount of preferably 0.1 mass% or less, more preferably 500 ppm by mass or less, and most preferably 100 ppm by mass or less based on the entire composition.

The composition according to the present disclosure may further contain acetylene in addition to HFO-1132 and the additional compounds.

### 2. Method for Producing Composition of Present

### Disclosure

The present inventors found that additional compounds may be mixed into HFO-1132 during storage, transport, and equipment operation of refrigerants containing HFO-1132. After further diligent examination, the present inventors found that, through purification methods such as rectification, alkali cleaning, and adsorption, the mixing ratio of these additional compounds can be adjusted to 1 mass% or less, preferably 0.5 mass% or less, based on the composition.

As the above purification methods, methods by adsorption and distillation are suitably used. For example, in the case of adsorption, activated carbon, molecular sieves, silica gel, metal organic frameworks (MOFs), etc. are used. The mixing ratio of these additional compounds can be adjusted by making a selection in consideration of the molecular size of the additional compounds to be adjusted in the target composition and the strength of their interaction with the adsorbent. Also, in the case of distillation, they can be separated by a normal distillation column. For example, by using a distillation column with the required number of theoretical stages of about 10 stages to about 200 stages, and by performing distillation under a pressure of normal pressure to about 2 MPaG, if necessary, these additional compounds can be concentrated at the top of the column or at the bottom of the column to obtain HFO-1132(E) with a decreased mixing ratio.

### 3. Heat Transfer Medium

The composition according to the present disclosure has a low GWP and sufficient stability, and can be used as a heat transfer medium.

In the case where the composition according to the present disclosure is used as a heat transfer medium, it may also be used as a refrigerant or component of a refrigerant with a lower GWP, replacing conventionally used refrigerants such as R22, R12, R134a, R410A, R407C, R404A, R507A, R502, etc.

The composition according to the present disclosure used as a heat transfer medium may contain at least one other component. The composition according to the present disclosure can be further mixed with at least a refrigeration oil to thereby obtain a working fluid for a refrigerating machine (the composition according to the present disclosure in this case is referred to as "refrigerant composition according to the present disclosure").

The refrigerant composition according to the present disclosure may comprise at least one of the following other components, if necessary. The other components are not limited, and specific examples thereof include water, tracers, ultraviolet fluorescent dyes, stabilizers, and polymerization inhibitors.

When the refrigerant composition according to the present disclosure is used as a working fluid in a refrigerating machine, it is generally used as a mixture with at least a refrigeration oil. Therefore, it is preferable that the refrigerant composition according to the present disclosure does not substantially comprise a refrigeration oil. Specifically, in the refrigerant composition according to the present disclosure, the content of the refrigeration oil based on the entire composition is preferably 0 to 1 mass%, and more preferably 0 to 0.1 mass%.

The refrigerant composition according to the present disclosure may contain a small amount of water. The water content of the refrigerant composition is preferably 0.1 mass% or less based on the entire refrigerant composition. A small amount of water contained in the refrigerant composition stabilizes double bonds in the molecules of unsaturated fluorocarbon compounds that can be present in the refrigerant, and makes it less likely that the unsaturated fluorocarbon compounds will be oxidized, thus increasing the stability of the refrigerant composition.

A tracer is added to the refrigerant composition according to the present disclosure at a detectable concentration such that when the refrigerant composition has been diluted, contaminated, or undergone other changes, the tracer can trace the changes.

The refrigerant composition according to the present disclosure may comprise a single tracer, or two or more tracers. Also, at least some of the above additional compounds can be utilized as tracers.

The tracer is not limited, and can be suitably selected from commonly used tracers.

Examples of tracers include hydrofluorocarbons, hydrochlorofluorocarbons, chlorofluorocarbons, hydrochlorocarbons, fluorocarbons, deuterated hydrocarbons, deuterated hydrofluorocarbons, perfluorocarbons, fluoroethers, brominated compounds, iodinated compounds, alcohols, aldehydes, ketones, and nitrous oxide (N₂O). The tracer is particularly preferably a hydrofluorocarbon, a hydrochlorofluorocarbon, a chlorofluorocarbon, a hydrochlorocarbon, a fluorocarbon, or a fluoroether.

The following compounds are preferable as the tracer.
FC-14 (tetrafluoromethane, CF₄)
HCC-40 (chloromethane, CH₃Cl)
HFC-23 (trifluoromethane, CHF₃)
HFC-134 (1,1,2,2-tetrafluoroethane, CHF₂CHF₂)
HFC-245fa (1, 1, 1, 3, 3-pentafluoropropane, CF₃CH₂CHF₂)
HFC-236fa (1, 1, 1, 3, 3, 3-hexafluoropropane, CF₃CH₂CF₃)
HFC-236ea (1,1,1,2,3,3-hexafluoropropane, CF₃CHFCHF₂)
HFC-227ea (1,1,1,2,3,3,3-heptafluoropropane, CF₃CHFCF₃)
HCFC-31 (chlorofluoromethane, CH₂ClF)
CFC-1113 (chlorotrifluoroethylene, CF₂=CClF)
HFE-125 (trifluoromethyl-difluoromethyl ether, CF₃OCHF₂)
HFE-134a (trifluoromethyl-fluoromethyl ether, CF₃OCH₂F)
HFE-143a (trifluoromethyl-methyl ether, CF₃OCH₃)
HFE-227ea (trifluoromethyl-tetrafluoroethyl ether, CF₃OCHFCF₃)
HFE-236fa (trifluoromethyl-trifluoroethyl ether, CF₃OCH₂CF₃)

The refrigerant composition according to the present disclosure may contain tracers in a total amount of about 10 parts per million (ppm) by weight to about 1000 ppm based on the entire refrigerant composition. The refrigerant composition according to the present disclosure may contain tracers in a total amount of preferably about 30 ppm to about 500 ppm, more preferably about 50 ppm to about 300 ppm, based on the entire refrigerant composition.

The refrigerant composition according to the present disclosure may comprise a single ultraviolet fluorescent dye, or two or more ultraviolet fluorescent dyes.

The ultraviolet fluorescent dye is not limited, and can be suitably selected from commonly used ultraviolet fluorescent dyes.

Examples of ultraviolet fluorescent dyes include naphthalimide, coumarin, anthracene, phenanthrene, xanthene, thioxanthene, naphthoxanthene, fluorescein, and derivatives thereof. The ultraviolet fluorescent dye is particularly preferably either naphthalimide or coumarin, or both.

The refrigerant composition according to the present disclosure may comprise a single stabilizer, or two or more stabilizers.

The stabilizer is not limited, and can be suitably selected from commonly used stabilizers.

Examples of stabilizers include nitro compounds, ethers, and amines. Examples of nitro compounds include aliphatic nitro compounds, such as nitromethane and nitroethane; and aromatic nitro compounds, such as nitro benzene and nitro styrene. Examples of ethers include 1,4-dioxane.

Examples of amines include 2,2,3,3,3-pentafluoropropylamine and diphenylamine.

Examples of stabilizers also include butylhydroxyxylene and benzotriazole.

The content of the stabilizer is not limited. Generally, the content of the stabilizer is preferably 0.01 to 5 mass%, and more preferably 0.05 to 2 mass%, based on the entire refrigerant composition.

The refrigerant composition according to the present disclosure may comprise a single polymerization inhibitor, or two or more polymerization inhibitors.

The polymerization inhibitor is not limited, and can be suitably selected from commonly used polymerization inhibitors.

Examples of polymerization inhibitors include 4-methoxy-1-naphthol, hydroquinone, hydroquinone methyl ether, dimethyl-t-butylphenol, 2,6-di-tert-butyl-p-cresol, and benzotriazole.

The content of the polymerization inhibitor is not limited. Generally, the content of the polymerization inhibitor is preferably 0.01 to 5 mass%, and more preferably 0.05 to 2 mass%, based on the entire refrigerant composition.

The composition according to the present disclosure can also be used as a working fluid for a refrigerating machine containing a refrigeration oil (this composition is referred to as "refrigeration oil-containing working fluid according to the present disclosure"). The refrigeration oil-containing working fluid according to the present disclosure comprises at least the refrigerant composition according to the present disclosure and a refrigeration oil, for use as a working fluid in a refrigerating machine. Specifically, the refrigeration oil-containing working fluid according to the present disclosure is obtained by mixing a refrigeration oil used in a compressor of a refrigerating machine with the refrigerant or the refrigerant composition. The refrigeration oil-containing working fluid generally comprises 10 to 50 mass% of refrigeration oil.

The refrigeration oil-containing working fluid according to the present disclosure may comprise a single refrigeration oil, or two or more refrigeration oils.

The refrigeration oil is not limited, and can be suitably selected from commonly used refrigeration oils. In this case, refrigeration oils that are superior in the action of increasing the miscibility with the mixture and the stability of the mixture, for example, are suitably selected as necessary.

The base oil of the refrigeration oil is preferably, for example, at least one member selected from the group consisting of polyalkylene glycols (PAG), polyol esters (POE), and polyvinyl ethers (PVE).

The refrigeration oil may further contain additives in addition to the base oil. The additive may be at least one member selected from the group consisting of antioxidants, extreme-pressure agents, acid scavengers, oxygen scavengers, copper deactivators, rust inhibitors, oil agents, and antifoaming agents.

A refrigeration oil with a kinematic viscosity of 5 to 400 cSt at 40°C is preferable from the standpoint of lubrication.

The refrigeration oil-containing working fluid according to the present disclosure may further optionally contain at least one additive. Examples of additives include compatibilizing agents described below.

The refrigeration oil-containing working fluid according to the present disclosure may comprise a single compatibilizing agent, or two or more compatibilizing agents.

The compatibilizing agent is not limited, and can be suitably selected from commonly used compatibilizing agents.

Examples of compatibilizing agents include polyoxyalkylene glycol ethers, amides, nitriles, ketones, chlorocarbons, esters, lactones, aryl ethers, fluoroethers, and 1,1,1-trifluoroalkanes. The compatibilizing agent is particularly preferably a polyoxyalkylene glycol ether.

The composition according to the present disclosure can be used in systems as a heat transfer medium. Examples of such systems include, without limitation, air conditioning units, freezers, refrigerators, heat pumps, water cooling units, flooded evaporator cooling units, direct expansion cooling units, centrifugal cooling units, mobile refrigerators, mobile air conditioning units, and combinations thereof.

### 4. Foaming Agent

The composition according to the present disclosure has a low GWP and sufficient stability, and can be used as a foaming agent. When the composition according to the present disclosure is used as a foaming agent, it has a lower GWP than conventional foaming agents.

In the case where the composition according to the present disclosure is used as a foaming agent, the composition may contain other foaming agents. The other foaming agents that can be used in combination are not limited, and examples thereof include halogenated hydrocarbons such as HFC227ea (1,1,1,2,3,3,3-heptafluoropropane), HCFO-1233zd (1-chloro-3,3,3-trifluoropropene), HFO-1234yf, and HFO-1234ze; and inert gases such as air, nitrogen, and carbon dioxide.

The composition according to the present disclosure may contain water. The addition of water is preferable because carbon dioxide gas is produced during foaming and the carbon dioxide gas contributes to foaming. However, addition of too much water may degrade the heat insulating performance, etc., of the foamed product.

The content of water is normally about 60 mass% or less based on the entire composition (100 mass%). When the content is within this range, highly heat insulating foamed products can be produced more reliably.

The composition according to the present disclosure may also contain a decomposition inhibitor, if necessary. The decomposition inhibitor is not limited, and preferred examples thereof include nitro compounds such as nitrobenzene and nitromethane; aromatic hydrocarbons such as α-methylstyrene and p-isopropenyltoluene; aliphatic unsaturated hydrocarbons such as isoprene and 2,3-dimethylbutadiene; epoxy compounds such as 1,2-butylene oxide and epichlorohydrin; phenolic compounds such as p-t-butylcatechol and 2,6-di-t-butyl-p-cresol; and chloroacetic acid ester compounds such as chloroacetic acid isopropyl ester.

The content of the decomposition inhibitor can be set as appropriate depending on the type of decomposition inhibitor, etc., and is normally about 0.05 to 5 mass% based on the entire composition (100 mass%).

### 5. Propellant

The composition according to the present disclosure has a low GWP and sufficient stability, and can be used as a propellant. When the composition according to the present disclosure is used as a propellant, it has a lower GWP than conventional propellants, and there can be provided a propellant that does not substantially contribute to global warming.

In the case where the composition according to the present disclosure is used as a propellant, the composition may contain other compounds, if necessary.

The other compounds are not limited, and examples thereof include HFC134a, HFC227ea (1,1,1,2,3,3,3-heptafluoropropane), HFO-1234yf, and HFO-1234ze.

In the case where the composition according to the present disclosure is used as a propellant, such a propellant can be blended into an aerosol. The aerosol is not limited, and examples thereof include various industrial aerosols such as contact cleaners, dusters, lubricant sprays, and other sprayable compositions; consumer aerosols such as personal care products, household products, medical aerosols, and automotive products. In the case of medical aerosols, other components may be further contained, such as drugs (beta agonists, corticosteroids, other drugs, etc.), surfactants, solvents, other propellants, flavoring agents, and excipients.

The embodiments are described above; however, it will be understood that various changes in forms and details can be made without departing from the spirit and scope of the claims.

### Examples

The present disclosure is described in more detail below with reference to Examples. However, the present disclosure is not limited to the Examples.

### (Example 1)

Four types of haloolefin compositions containing HFO-1132(E) and monofluoroacetylene HC*CF (* is a triple bond) were prepared in which the content of monofluoroacetylene was 1 ppm by weight, 10 ppm by weight, 100 ppm by weight, and 1000 ppm by weight based on HFO-1132(E), respectively.

The above HFO-1132(E) was produced by, for example, the method as shown in Japanese Patent Laid-Open No. 2019-196312. The HF produced at this time was deacidified by a water washing column and an alkali column with an aqueous NaOH solution. The obtained haloolefin compositions may contain 0 to 100 ppm by mass of water and 0 to 10 mol ppm of oxygen used in the production of HFO-1132 (E) .

### (Stability Test of Haloolefin)

A stability test of haloolefin was conducted on each of the haloolefin compositions obtained in the above Examples and Comparative Examples as follows. To a glass tube (ID 8 mmΦ × OD 12 mmΦ × L 300 mm) that had been melt-sealed on one side, a haloolefin composition was added such that the content of haloolefin was 0.01 mol. The tube was tightly closed by melt sealing. This tube was left to stand still in a thermostatic chamber under an atmosphere of 150°C and kept in this state for one week. Thereafter, the tube was taken out of the thermostatic chamber, cooled, and the acid content of the gas in the tube was analyzed to evaluate the stability of the haloolefin.

The analysis of acid content in the gas was conducted as follows. For the tube after the above cooling, liquid nitrogen was used to completely solidify the gas remaining in the tube. Thereafter, the tube was opened and the gas was collected in a tedlar bag by gradual defrosting. 5 g of pure water was injected into this tedlar bag to extract the acid content into the pure water while allowing it to come in good contact with the collected gas. The extraction liquid was detected by ion chromatography to determine the content (ppm by weight) of fluoride ions (F⁻).

Table 1 shows the test results.

**[Table 1]**

| No. | Example/ Comparative Example | Type of additional compound | Amount of compound (wt%) | Amount of moisture (ppm by weight) | Gas phase oxygen concentration Molppm | Content of acid content (ppm by weight) |
|---|---|---|---|---|---|---|
| | | | | | | F- |
| 1 | Example 1 | Monofluoroacetylene | 1 | 0(N.D.) | 10 | <1 |
| 2 | Example 1 | | 10 | 0(N.D.) | 10 | <1 |
| 3 | Example 1 | | 100 | 0(N.D.) | 10 | <1 |
| 4 | Comparative Example 1 | | 1000 | 0(N.D.) | 10 | <1 |
| 5 | Example 1 | | 1 | 10 | 10 | <1 |
| 6 | Example 1 | | 10 | 10 | 10 | 2 |
| 7 | Example 1 | | 100 | 10 | 10 | 35 |
| 8 | Comparative Example 1 | | 1000 | 10 | 10 | 610 |
| 9 | Comparative Example 1 | | 1 | 10 | 3400 | 1 |
| 10 | Comparative Example 1 | | 10 | 10 | 3400 | 4 |
| 11 | Comparative Example 1 | | 100 | 10 | 3400 | 54 |
| 12 | Comparative Example 1 | | 1000 | 10 | 3400 | 770 |
| 13 | Example 1 | | 1 | 100 | 10 | <1 |
| 14 | Example 1 | | 10 | 100 | 10 | 8 |
| 15 | Example 1 | | 100 | 100 | 10 | 87 |
| 16 | Comparative Example 1 | | 1000 | 100 | 10 | 920 |

### (Example 2)

Four types of haloolefin compositions containing 1,2-difluoroethylene (hereinafter abbreviated as "HFO-1132(E)") and formyl fluoride HCOF were prepared in which the content of formyl fluoride was 1 ppm by weight, 10 ppm by weight, 100 ppm by weight, and 1000 ppm by weight based on HFO-1132(E), respectively.

HCOF + H2O -> HCOOH + HF

Thus, the compositions may also contain formic acid produced by decomposition.

The above HFO-1132(E) was produced by, for example, the method as shown in Japanese Patent Laid-Open No. 2019-196312. The HF produced at this time was deacidified by a water washing column and an alkali column with an aqueous NaOH solution. The obtained haloolefin compositions may contain 0 to 100 ppm by mass of water and 0 to 10 mol ppm of oxygen used in the production of HFO-1132(E). Table 2 shows the measurement results.

**[Table 2]**

| No. | Example/ Comparative Example | Type of additional compound | Amount of compound (wt%) | Amount of moisture (ppm by weight) | Gas phase oxygen concentration Molppm | Content of acid content (ppm by weight) F- |
|---|---|---|---|---|---|---|
| 17 | Example 2 | Formyl fluoride | 1 | 0(N.D.) | 10 | <1 |
| 18 | Example 2 | | 10 | 0(N.D.) | 10 | <1 |
| 19 | Example 2 | | 100 | 0(N.D.) | 10 | <1 |
| 20 | Comparative Example 2 | | 1000 | 0(N.D.) | 10 | <1 |
| 21 | Example 2 | | 1 | 10 | 10 | <1 |
| 22 | Example 2 | | 10 | 10 | 10 | 3 |
| 23 | Example 2 | | 100 | 10 | 10 | 57 |
| 24 | Comparative Example 2 | | 1000 | 10 | 10 | 790 |
| 25 | Comparative Example 2 | | 1 | 10 | 3400 | 1 |
| 26 | Comparative Example 2 | | 10 | 10 | 3400 | 5 |
| 27 | Comparative Example 2 | | 100 | 10 | 3400 | 65 |
| 28 | Comparative Example 2 | | 1000 | 10 | 3400 | 850 |
| 29 | Example 2 | | 1 | 100 | 10 | <1 |
| 30 | Example 2 | | 10 | 100 | 10 | 9 |
| 31 | Example 2 | | 100 | 100 | 10 | 92 |
| 32 | Comparative Example 2 | | 1000 | 100 | 10 | 950 |

### (Example 3)

Four types of haloolefin compositions containing 1,2-difluoroethylene (hereinafter abbreviated as "HFO-1132(E)") and 1,2,3-trifluorocyclopropane were prepared in which the content of 1,2,3-trifluorocyclopropane was 1 ppm by weight, 10 ppm by weight, 100 ppm by weight, and 1000 ppm by weight based on HFO-1132(E), respectively.

The above HFO-1132(E) was produced by, for example, the method as shown in Japanese Patent Laid-Open No. 2019-196312. The HF produced at this time was deacidified by a water washing column and an alkali column with an aqueous NaOH solution. The obtained haloolefin compositions may contain 0 to 100 ppm by mass of water and 0 to 10 mol ppm of oxygen used in the production of HFO-1132(E). Table 3 shows the measurement results.

**[Table 3]**

| No. | Example/ Comparative Example | Type of additional compound | Amount of compound (wt%) | Amount of moisture (ppm by weight) | Gas phase oxygen concentration Molppm | Content of acid content (ppm by weight) |
|---|---|---|---|---|---|---|
| | | | | | | F- |
| 33 | Example 3 | 1,2,3-Trifluorocyclopropane | 1 | 0(N.D.) | 10 | <1 |
| 34 | Example 3 | | 10 | 0(N.D.) | 10 | <1 |
| 35 | Example 3 | | 100 | 0(N.D.) | 10 | <1 |
| 36 | Comparative Example 3 | | 1000 | 0(N.D.) | 10 | <1 |
| 37 | Example 3 | | 1 | 10 | 10 | <1 |
| 38 | Example 3 | | 10 | 10 | 10 | 2 |
| 39 | Example 3 | | 100 | 10 | 10 | 27 |
| 40 | Comparative Example 3 | | 1000 | 10 | 10 | 480 |
| 41 | Comparative Example 3 | | 1 | 10 | 3400 | 1 |
| 42 | Comparative Example 3 | | 10 | 10 | 3400 | 4 |
| 43 | Comparative Example 3 | | 100 | 10 | 3400 | 40 |
| 44 | Comparative Example 3 | | 1000 | 10 | 3400 | 600 |
| 45 | Example 3 | | 1 | 100 | 10 | <1 |
| 46 | Example 3 | | 10 | 100 | 10 | 3 |
| 47 | Example 3 | | 100 | 100 | 10 | 51 |
| 48 | Comparative Example 3 | | 1000 | 100 | 10 | 720 |

As shown in these tables, in the case where the additional compounds were controlled at 10 to 100 ppm or less, decomposition was suppressed when both moisture and oxygen were at low concentrations. However, decomposition was accelerated when the concentration of these additional compounds was increased in the coexistence of moisture and oxygen. In the samples where the additional compounds were present at high concentrations, degradation proceeded rapidly, and some sealed glass tubes were damaged, causing the test to be redone.

## Claims

1. A composition comprising:
1,2-difluoroethylene (HFO-1132); and
at least one compound selected from the group consisting of: a fluoroacetylene represented by CX*CR¹, wherein * is a triple bond, R¹ is a monovalent group represented by CₙX²ₘ, where n is 0, 1, or 2, m = 2n + 1, and X and X¹ are the same or different and are H or F, and at least one of X and X¹ is F; a carboxylic acid fluoride represented by R²COF, wherein R² is a monovalent group represented by CₒX²ₚ, where o is 0 or 1, p = 2o + 1, and X² is H or F; a cyclic alkane represented by C_{q}HᵣFₛ, wherein q is 3, 4, or 5, r and s are integers of 0 or more and 2q or less that are the same as or different from each other, and r + s < 2q; and formic acid.

2. The composition according to claim 1, comprising the compounds in a total amount of 0.1 mass% or less based on the composition.

3. The composition according to claim 1 or 2, which is a heat transfer medium, a foaming agent, or a propellant.
